Europäisches Patentamt

European Patent Office

Office européen des brevets

(11) Publication number: **0 427 474 A2**

# EUROPEAN PATENT APPLICATION

(21) Application number: 90312026.9

(22) Date of filing: 02.11.90

(30) Priority: 09.11.89 US 433699

(43) Date of publication of application:
15.05.91 Bulletin 91/20

(84) Designated Contracting States:
DE ES FR IT SE

(51) Int. Cl.⁵: **A61M 16/06, A62B 7/00**

(71) Applicant: **SMITHS INDUSTRIES MEDICAL
SYSTEMS INC.
Kit Street, P.O. Box 724
Keene, New Hampshire 03431-0724(US)**

(72) Inventor: **Hollister, William Hermann
P.O. Box 458
East Sullivan, New Hampshire 03445(US)**
Inventor: **Carruthers, Cary Donald
185 Bowkerville Road
Troy,New Hampshire 03465(US)**

(74) Representative: **Flint, Jonathan McNeill
SMITHS INDUSTRIES PUBLIC LIMITED
COMPANY 765 Finchley Road
London NW11 8DS(GB)**

(54) Face masks and face masks components.

(57) A cushion member 10 for a face mask comprises a water activated, hydrophilic, open cell foam. The foam is formed with a urethane prepolymer having a functionality of less than 2, the prepolymer being toluene diisocyanate prepolymer. The specific gravity of the cushion member 10 is between 0.1 and 0.2 with a durometer hardness of 5 to 2 on the Shore A scale. The average cell diameter is 0.020 to 0.127mm. The cushion member 10 is covered by a thin skin between 0.025 and 0.127mm thick of surgical latex, acrylic latex, PVC, urethane, plastisol, nitrile or latex. The foam is made by combining an aqueous mixture of water and surfactant with a urethane prepolymer for the hydrophilic polyurethane foam.

FIG.5

EP 0 427 474 A2

## FACE MASKS AND FACE MASK COMPONENTS

The present invention relates to cushion members for a face mask.

Face masks for the administration to a wearer of gases, such as oxygen or anaesthesia gases, in general, comprise two main components: a cushion member and a dome-shaped semi-rigid faceplate to which the cushion member is attached. The faceplate is provided with a gas inlet.

The cushion member should form an essentially leak-free seal about the face of the wearer to minimize escape of the gas being administered into the atmosphere. Therefore, due to the great variation in facial anatomy from person to person, it is highly desirable that the cushion member comprise a resilient, flexible material which is sufficiently soft so as to conform to such variations and to form an adequate seal about the face of the wearer. The cushion member is also relatively impermeable to the gas being administered.

The faceplate is preferably made of a transparent material such that the user may observe the wearer's nose and mouth, and should have sufficient rigidity that the user can apply pressure thereon to cause the cushion member to form a seal about the wearer's face. It is highly desirable that an adequate seal be attained with a minimum of pressure in order to minimize discomfort to the wearer. It is also desirable that the user should be able to achieve a secure grip on the faceplate in order to achieve and maintain an adequate seal about the face of the wearer. Moreover, the faceplate should be constructed such that the discomfort to the wearer and the user is minimized, particularly where the face mask is worn over an extended period of time.

It is also desirable that such face masks are designed for single use only to prevent patient-to-patient cross-contamination and infection, it is therefore required that the face mask is made of materials that are economical to manufacture and are readily disposable.

A wide variety of face masks for the administration of gases are known in the art. See for example, US Patent Number Des. 300,473; US Patent No. 4,062,357 and US Patent No. 4,803,981. Most such face masks suffer one or more disadvantages, such as an insufficient degree of contact between the face mask and the wearer's face, causing unnecessary discomfort to the wearer or the user, or the design involves time consuming manufacturing procedures. Furthermore, the faceplate typically found in face masks is smooth and does not provide a means for the user to achieve a secure grip on the mask.

A wide variety of face masks which provide breathing protection, e.g., against the inhalation of dust and other particulate matter, are also known in the art. See, for example, US Patent Nos. 4,195,629; 4,619,948; 4,296,746; and 4,037,593. Such face masks have disadvantages similar to those described above.

It is an object of the present invention to provide an improved form of face mask which alleviates at least some of the above-mentioned disadvantages.

According to one aspect of the present invention there is provided a cushion member for a face mask comprising a water activated, hydrophilic polyurethane foam.

The hydrophilic polyurethane foam is preferably an open cell foam and may be formed with a urethane prepolymer having a functionality of less than 2. The prepolymer may be toluene diisocyanate prepolymer.

The cushion member preferably has a specific gravity between 0.1 and 0.2 and a durometer hardness between 5 and 2 on the shore A scale. The average cell diameter is between 0.025 and 0.127mm.

The cushion member may be provided with a thin essentially gas impermeable skin. The skin may be between 0.025 and 0.127mm thick. The thin skin may comprise at least one of surgical latex, acrylic latex, PVC, urethane, plastisol, nitile or latex.

According to a second aspect of the present invention there is provided a method of forming a foam cushion member for a face mask, characterised in that the method includes the steps of: (a) forming an aqueous mixture comprising water and surfactant; (b) combining the aqueous mixture obtained in step (a) with a urethane prepolymer for a hydophilic polyurethane foam under conditions to enable the formation of a foaming mess; and (c) forming the foaming mass into the cushion member.

The aqueous mixture preferably further comprises at least one filler and lecithin.

A faceplate for a face mask in accordance with the present invention, will be described, by way of example, with reference to the accompanying drawings, in which:

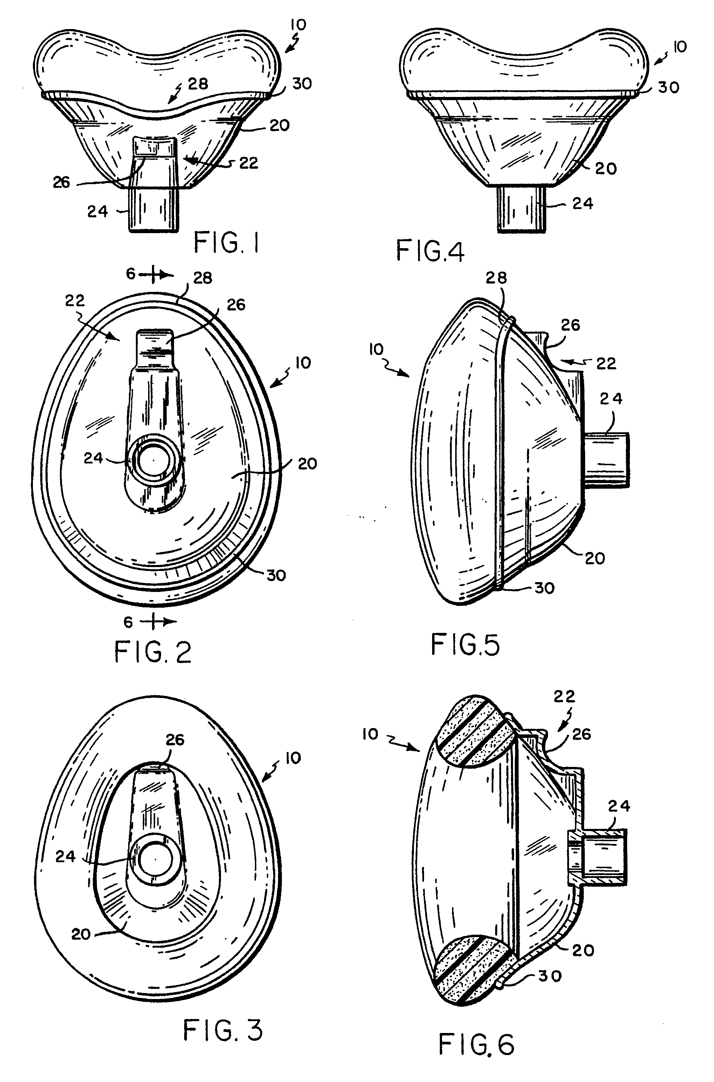

Figure 1 is a top plan view of the face mask;

Figure 2 is a front elevational view of the mask shown in Figure 1;

Figure 3 is a rear elevation view of the mask shown in Figure 1;

Figure 4 is a bottom plan view of the mask of Figure 1;

Figure 5 is a side elevational view of the mask shown in Figure 1, the opposite side being a mirror image thereof; and

Figure 6 is a section taken along line 6-6 of Figure 2.

The mask comprises two parts: a cushion member 10 and a faceplate 20.

The cushion member 10 comprises a water activated hydrophilic polyurethane foam which is contoured to mould around the human face. Preferred polyurethane foam cushion members in accordance with the present invention are soft and compliant, having a low density with a specific gravity from about 0.1 to 0.2. A specific gravity of about 0.1 is especially preferred.

The cushion member may be formed by a method comprising:

(a) forming an aqueous mixture comprising water and surfactant;

(b) combining the aqueous mixture obtained in (a) with a prepolymer for the hydrophilic polyurethane foam under conditions to enable the formation of a foaming mass; and,

(c) forming the foaming mass into the cushion member 10.

It is preferred that the aqueous mixture and the prepolymer be mixed as they are poured into an appropriate mould from a mixing head set at high speed, e.g., 1,000 to 2,000 rpm or higher. Although the above method is preferred for forming the cushion members of the present invention, other methods known to the skilled artisan can be used to form the cushion members.

Urethane prepolymers for use in the present invention preferably have a functionality of less than 2. Toluene diisocyanate prepolymers are preferred. Such prepolymers include Hypol 2002 (W. R. Grace, Inc.), and Trepol A62 (Rynel, Inc.).

In preferred embodiments, the water-activated hydrophilic polyurethane foam is a very fine structured, open celled foam. In order to reduce the permeability of such foam materials to the gas being administered, additives which reduce permeability may be incorporated into the foam. Such ingredients include calcium carbonate, acrylic latex, mica and talc. Alternatively, the cushion member 10 may be provided with a thin skin, not shown, comprising an essentially gas-impermeable composition, such as surgical latex, acrylic latex, PVC, urethane, plastisol, nitrile rubber latex, or a mixture of one or more of the above. Skin thickness is typically about 0.025mm to 0.127mm.

Surfactants are used to reduce the cell structure to produce a super soft, very fine celled foam. As used herein "super soft" means a durometer hardness of about 5 to 2 or less on the Shore A scale and "very fine celled" means an average cell diameter of about 0.025mm to 0.127mm. Surfactants for use in the present invention include Brij 72 (ICN America, Inc.), Pluronic L62 (BASF Wynodotte, Inc.) and Dispex N40 (Allied Colloids, Inc.).

The precise composition of the polyurethane foam forming the claimed cushion member is dictated primarily by the desired characteristics of the cushion member 10, such as softness, flexibility and resiliency. Other components often found in urethane polymers may optionally be included in the cushion members. Such ingredients may be added at any appropriate stage in the formation of the cushion members 10 of the present invention and include, emulsifiers, whiteners, inert fillers and so forth. These optional ingredients will be employed in amounts to provide the desired function as is known by the skilled artisan.

Emulsifiers may be optionally included to achieve easier mixing of the aqueous mixture and the prepolymer and further to reduce foam cell size and enhance softness. Lecithin is a preferred emulsifier. Whiteners, such as titanium dioxide may also be used to achieve a cushion member 10 having an enhanced appearance. Fillers may also be used in forming the cushion members of the present invention to tailor the degree of stiffness and body.

The faceplate 20 is provided with a thumb grip 22 which is positioned in the area of the faceplate 20 to lie over the bridge of the nose and forwardly of a member 24 for connecting the face mask to a gas source, not shown.

The thumb grip 22 comprises a trapezoidal member which extends forwardly from the gas inlet tube 24 towards the periphery of the faceplate 20. The thumb grip 22 is provided with the depression 26 for accepting the thumb. The thumb grip 22 enables the user to achieve a secure grasp on the mask, thereby facilitating application of the mask to the face of the wearer.

In order to provide additional comfort for the wearer, the faceplate 20 is designed to accommodate the bridge of the wearer's nose by means of a nose bridge relief 28, so that excessive pressure is not transferred to that area when pressure is applied to the mask to form a seal about the wearer's face. The perimeter of the faceplate 20 in the area of the bridge of the nose is contoured in the region of the relief 28 to conform to the wearer's face, to reduce the pressure transferred to the bridge of the nose. The thumb grip 22 is positioned immediately rearwardly of the nose bridge relief 28.

The faceplate also has a beaded edge 30 which extends around the periphery of the faceplate and provides a smooth leading edge. The beaded edge 30 is provided in order to prevent the fingers of a user, e.g., an anaesthetist, from encountering a sharp leading edge as is found in conventional face masks.

The faceplate 20 is preferably transparent and is sufficiently rigid that adequate pressure can be applied to the mask by the user to obtain an essentially gas-tight seal about the face of the wearer. Preferred materials for the faceplate 20 include PVC, polyurethane, K-Resin and Clear ABS. Particularly preferred faceplates for use in the present invention are injection-moulded of a semi-rigid, clear thermoplastic, such as urethane or PVC.

The faceplate 20 is adhered to the cushion member 10 by an adhesive or by the intrinsic adherent qualities of the polyurethane foam cushion. It is preferable in most instances to use an adhesive such as UV curable acrylics; hot melts; PVC dissolved in Tetra Hydro Furan; epoxides, cyanoacrylate and urethane adhesives. A preferred adhesive is Litetak 376 (made by Loctite Corp.), a UV curable acrylic adhesive.

It is anticipated that the cushion members of the present invention may also be used for mask applications which do not require an essentially gas-impermeable cushion member. For example, masks for the filtration of particles, such as dust, or the filtration of gases would not require a gas-impermeable cushion member but would benefit from use of a cushion member in accordance with the present invention so as to provide a mask which better conforms to the wearer's face, and thus, provides enhanced exclusion of particles or gases.

In such masks, the faceplate may be formed from a transparent and semi-rigid plastic material which is provided with ventilation means. The faceplate may also be formed from an appropriate filtering material as are known to those skilled in the art.

The invention will be further understood with reference to the following examples, which are exemplary in nature and are not meant to limit the scope of the invention.

## Example I - Preparation of Cushion Member

A polyurethane prepolymer, Trepol Isocyanate Capped Polyol, available from Rynel Inc., was used to prepare a cushion member similar to that shown in the figures.

The aqueous phase (in percent weight) comprised: water (23.65%); Dispex N40, a surfactant (0.189%); titanium dioxide, a whitener (6.66 percent weight); Walastonite (35.65%); a mineral (CaCO) filler; and EG-HS-3 - 2.5% (33.65%). The EG-HS-3 is Emulgade (2.5% surfactant plus $HS_3$ Lecithin). The Emulgade surfactant is from Heinkle, Inc. and the $HS_3$ Lecithin is from Alcolec, Inc.

The prepolymer and aqueous phase were mixed at a ratio of 1.5 parts aqueous mixture to 1.0 part prepolymer and poured into the mould as described below to form the cushion member 10.

The mould was a one piece silicone elastomer mould which defines the shape of the cushion member. Prepolymer and aqueous phases were joined in a high speed mixing head and mixed just prior to being dispensed into the silicon mould. The dispensing head precisely metered the shot size in grams for the mould. About 40 grams is typically used to form a cushion member for an adult mask. After the precise amount by weight of mixed prepolymer and aqueous phase were dispensed into the silicone mould, a flat top plate was placed on top of the mould, the foam was allowed to rise to meet the top plate, excess air being expelled through the vent holes in the top plate. After a suitable curing time, the top plate was removed and the urethane polyurethane cushion was pulled from the mould and allowed to dry.

## Example II - Coating the Cushion Member

The cushion member formed in Example 1 above, after drying and curing, was dipped in acrylic latex consisting of a Ucar 154 mixture with 300 parts Ucar 154 to 100 parts Ucar 365 and the latex was allowed to dry for about 20 minutes. Ucar is available from Union Carbide Corporation.

## Example III - Forming the Faceplate with Thumb-Grip and Attaching the Cushion Member

A faceplate provided with a thumb grip and nose bridge relief similar to that shown in the figures was made by injection moulding PVC. The shape of the faceplate was machined into the halves of the injection moulds themselves. The molten material was injected into the mould and allowed to cool to make the clear faceplate member.

The faceplate was attached to the cushion member formed in Examples I and II above by means of Litetak 376, Loctite Corp.

In is understood that the Examples and embodiments described herein are for illustrative purposes only and that various modifications or changes, in light thereof, that will be suggested to persons skilled in the art are to be included in the spirit and purview of this application and the scope of the approved claims.

## Claims

1. A cushion member for a face mask, characterised in that the cushion member (10) comprises a water activated, hydrophilic polyurethane foam.

2. A cushion member according to Claim 1, characterised in that the water activated, hydrophilic polyurethane foam is an open cell foam.

3. A cushion member according to Claims 1 or 2, characterised in that the polyurethane foam is formed with a urethane prepolymer having a functionality of less than 2.

4. A cushion member ac.cording to Claim 3, characterised in that the prepolymer is a toluene diisocyanate prepolymer.

5. A cushion member according to any one of the preceding claims, characterised in that the cushion member (10) has a specific gravity between 0.1 and 0.2.

6. A cushion member according to any one of the preceding claims, characterised in that the cushion member (10) has a durometer hardness of between 5 and 2 on the Shore A scale.

7. A cushion member according to any one of the preceding claims, characterised in that the average cell diameter is between 0.025 and 0.127mm.

8. A cushion member according to any one of the preceding claims, characterised in that the cushion member (10) provided with a thin essentially gas impermeable skin.

9. A cushion member according to Claim 9, characterised in that the skin is between 0.025 and 0.127mm thick.

10. A cushion member according to Claim 9 or 10, characterised in that the thin skin comprises at least one of surgical latex, acrylic latex, PVC, urethane, plastisol, nitrile or latex.

11. A method of forming a foam cushion member for a face mask, characterised in that the method includes the steps of:

    (a) forming an aqueous mixture comprising water and surfactant;

    (b) combining the aqueous mixture obtained in step (a) with a urethane prepolymer for a hydrophilic polyurethane foam under conditions to enable the formation of a foaming mass; and

    (c) forming the foaming mass into the cushion member.

12. The method of Claim 12, characterised in that the aqueous mixture further comprises at least one filler an lecithin.

FIG. 1

FIG.4

FIG. 2

FIG.5

FIG. 3

FIG.6